(19) Europäisches Patentamt / European Patent Office / Office européen des brevets

(11) **EP 4 092 376 A1**

(12) **EUROPEAN PATENT APPLICATION**
published in accordance with Art. 153(4) EPC

(43) Date of publication:
**23.11.2022 Bulletin 2022/47**

(21) Application number: **20913106.9**

(22) Date of filing: **12.10.2020**

(51) International Patent Classification (IPC):
$G01B\ 9/02^{(2022.01)}$     $G01B\ 11/24^{(2006.01)}$
$C12M\ 1/34^{(2006.01)}$

(52) Cooperative Patent Classification (CPC):
**C12M 1/34; G01B 9/02; G01B 11/24**

(86) International application number:
**PCT/JP2020/038539**

(87) International publication number:
**WO 2021/145035 (22.07.2021 Gazette 2021/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.01.2020 JP 2020006381**

(71) Applicant: **FUJIFILM Corporation
Tokyo 106-8620 (JP)**

(72) Inventor: **MATSUBARA, Kenta
Ashigarakami-gun, Kanagawa 258-8577 (JP)**

(74) Representative: **Klunker IP
Patentanwälte PartG mbB
Destouchesstraße 68
80796 München (DE)**

(54) **INFORMATION PROCESSING DEVICE, OPERATION METHOD FOR INFORMATION PROCESSING DEVICE, AND OPERATION PROGRAM FOR INFORMATION PROCESSING DEVICE**

(57) An information processing apparatus that executes processing of obtaining, from an interference fringe image that is a two-dimensional distribution of intensity of interference fringes of object light as illumination light diffracted by an object to be observed and reference light without passing through the object to be observed, a phase difference image that is a two-dimensional distribution of a phase difference between the object light and the reference light, and obtaining a shape of the object to be observed based on the phase difference image includes at least one processor configured to acquire object-related information regarding the object to be observed, read out, from a storage unit in which the object-related information and a shape profile indicating the shape of the object to be observed are stored in association with each other, the shape profile corresponding to the acquired object-related information, and perform phase connection with respect to the phase difference image with reference to the read-out shape profile.

FIG. 14

**Description**

1. Field of the Invention

A technique of the present disclosure relates to an information processing apparatus, a method for operating an information processing apparatus, and an operation program for an information processing apparatus.

2. Description of the Related Art

As a method of measuring a micro surface rugged shape of an object to be observed, a light interference measurement method is known. In the light interference measurement method, first, from an interference fringe image that is a two-dimensional distribution of intensity of interference fringes of object light as illumination light diffracted by an object to be observed and reference light without passing through the object to be observed, a phase difference image that is a two-dimensional distribution of a phase difference between the object light and the reference light is obtained. Then, a method that obtains, as the shape of the object to be observed, a height of the object to be observed along an irradiation direction of the illumination light based on the obtained phase difference image.

A pixel value of the phase difference image is obtained as a function of arctan. For this reason, the pixel value of the phase difference image is obtained to be folded in a range of $-\pi$ to $\pi$ (wrapping) that is a range of arctan. For this reason, an actual phase difference is hardly known only with the pixel value, such as a case where the pixel value to be obtained is $\pi/4$ even though an actual phase difference is $5\pi/4$. Accordingly, as the pixel value is obtained to be folded in the range of $-\pi$ to $\pi$, in the phase difference image, the pixel value may have a phase jump of about $\pm 2\pi$ in places.

In a case where there is such a phase jump, it is not possible to accurately obtain the height of the object to be observed. Accordingly, before obtaining the height of the object to be observed, processing of connecting the phases by adding or subtracting $2\pi$ to a portion of the phase difference image where there is a phase jump is executed. Such processing of connecting the phases is referred to as phase connection (or referred to as phase unwrapping). Since the phase connection is performed while repeating trial and error by selecting an optimum path of the phase connection in the phase difference image or the like, an appropriate time is spent.

JP2006-284186A describes a technique that actually measures a rough shape of an object to be observed and performs phase connection with respect to a phase difference image with reference to a measurement result. According to the technique described in JP2006-284186A, since the measurement result of the rough shape of the object to be observed is an important clue, it is possible to reduce a processing time of the phase connection compared to a case where the phase connection is performed without any information.

SUMMARY OF THE INVENTION

In JP2006-284186A, there is a need for a mechanism for measuring the rough shape of the object to be observed. An excess measurement time is spent.

An object of the technique of the present disclosure is to provide an information processing apparatus, a method of operating an information processing apparatus, and an operation program for an information processing apparatus capable of reducing a processing time of phase connection without needing for a special mechanism for measuring a rough shape of an object to be observed and without spending an excess measurement time.

To attain the above-described object, there is provided an information processing apparatus of the present disclosure that executes processing of obtaining, from an interference fringe image that is a two-dimensional distribution of intensity of interference fringes of object light as illumination light diffracted by an object to be observed and reference light without passing through the object to be observed, a phase difference image that is a two-dimensional distribution of a phase difference between the object light and the reference light, and obtaining a shape of the object to be observed based on the phase difference image, the information processing apparatus comprising at least one processor configured to acquire object-related information regarding the object to be observed, read out, from a storage unit in which the object-related information and a shape profile indicating the shape of the object to be observed are stored in association with each other, the shape profile corresponding to the acquired object-related information, and perform phase connection with respect to the phase difference image with reference to the read-out shape profile.

It is preferable that the at least one processor is configured to extract a presence region of the object to be observed from the interference fringe image, and selectively perform the phase connection with respect to the presence region.

It is preferable that the at least one processor is configured to perform control of displaying a calculation result of the shape of the object to be observed.

It is preferable that the at least one processor is configured to generate a reproduction image representing any tomographic plane of the object to be observed from the interference fringe image, and display the calculation result of the shape of the object to be observed on the reproduction image in a superimposed manner.

It is preferable that the shape of the object to be observed is a height of the object to be observed along an irradiation direction of the illumination light.

It is preferable that the object to be observed is a cell during culture. In this case, it is preferable that the at least one processor is configured to acquire culture surface height-related information regarding a height from a bottom surface to a culture surface of a culture vessel of the cell.

It is preferable that the object-related information is a type of the cell and a culture condition of the cell. In this case, it is preferable that the culture condition includes at least any one of the number of days of culture, a type of a culture medium, a temperature of a culture environment, or a carbon dioxide concentration of the culture environment.

There is provided a method for operating an information processing apparatus of the present disclosure that executes processing of obtaining, from an interference fringe image that is a two-dimensional distribution of intensity of interference fringes of object light as illumination light diffracted by an object to be observed and reference light without passing through the object to be observed, a phase difference image that is a two-dimensional distribution of a phase difference between the object light and the reference light, and obtaining a shape of the object to be observed based on the phase difference image, the method comprising an acquisition step of acquiring object-related information regarding the object to be observed, a readout step of reading out, from a storage unit in which the object-related information and a shape profile indicating the shape of the object to be observed are stored in association with each other, the shape profile corresponding to the object-related information acquired in the acquisition step, and a phase connection step of performing phase connection with respect to the phase difference image with reference to the shape profile read out in the readout step.

There is provided an operation program for an information processing apparatus of the present disclosure that executes processing of obtaining, from an interference fringe image that is a two-dimensional distribution of intensity of interference fringes of object light as illumination light diffracted by an object to be observed and reference light without passing through the object to be observed, a phase difference image that is a two-dimensional distribution of a phase difference between the object light and the reference light, and obtaining a shape of the object to be observed based on the phase difference image, the operation program causing a computer to function as an acquisition unit that acquires object-related information regarding the object to be observed, a readout unit that reads out, from a storage unit in which the object-related information and a shape profile indicating the shape of the object to be observed are stored in association with each other, the shape profile corresponding to the object-related information acquired in the acquisition unit, and a phase connection unit that performs phase connection with respect to the phase difference image with reference to the shape profile read out in the readout unit.

According to the technique of the present disclosure, it is possible to provide an information processing apparatus, a method for operating an information processing apparatus, and an operation program for an information processing apparatus capable of reducing a processing time of phase connection without needing for a special mechanism for measuring a rough shape of an object to be observed and without spending an excess measurement time.

BRIEF DESCRIPTION OF THE DRAWINGS

Fig. 1 is a diagram showing a digital holography system.
Fig. 2 is a diagram showing a measurement apparatus.
Fig. 3 is a diagram showing states of object light and transmitted light near an imaging surface of an imaging element, and an interference fringe image.
Figs. 4A and 4B are diagrams illustrating composition of interference fringes, and specifically, Fig. 4A shows formation of bright portions of the interference fringes, and Fig. 4B shows formation of dark portions of the interference fringes.
Figs. 5A to 5D are diagrams showing a manner of obtaining an interference fringe image in the measurement apparatus, and specifically, Fig. 5A shows a case of a shift amount of 0, Fig. 5B shows a case of a shift amount of $\pi/2$, Fig. 5C shows a case of a shift amount of $\pi$, and Fig. 5D shows a case of a shift amount of $3\pi/2$.
Fig. 6 is a diagram showing formation of a phase difference image.
Fig. 7 is a diagram showing a height of a cell.
Fig. 8 is a block diagram showing a computer that configures an information processing apparatus.
Fig. 9 is a block diagram showing a CPU of the information processing apparatus.
Fig. 10 is a diagram showing an information input screen.
Fig. 11 is a diagram showing a shape profile table.
Fig. 12 is a diagram showing a culture surface height table.
Fig. 13 is a diagram showing details of a processing unit.
Fig. 14 is a diagram showing details of processing of a phase difference image generation unit and a phase connection unit.

Fig. 15 is a diagram illustrating phase connection.

Fig. 16 is a diagram showing a manner of actual phase connection.

Fig. 17 is a diagram showing details of processing of a height calculation unit.

Fig. 18 is a diagram showing a measurement result display screen.

Fig. 19 is a flowchart illustrating a processing procedure of the information processing apparatus.

Figs. 20A to 20C are diagrams illustrating the effects of the technique of the present disclosure, and specifically, Fig. 20A shows a case where a shape profile is not referred to, and Figs. 20B and 20C show a case where a shape profile is referred to.

Fig. 21 is a diagram showing another example of object-related information.

Fig. 22 is a diagram showing a second embodiment where a presence region of a cell is extracted and phase connection is selectively performed with respect to the presence region.

Fig. 23 is a diagram showing a third embodiment where a reproduction image is generated from an interference fringe image.

Fig. 24 is a diagram showing the outline of arithmetic processing by a generation unit.

Fig. 25 is a diagram showing a measurement result display screen of a third embodiment.

## DESCRIPTION OF THE PREFERRED EMBODIMENTS

[First Embodiment]

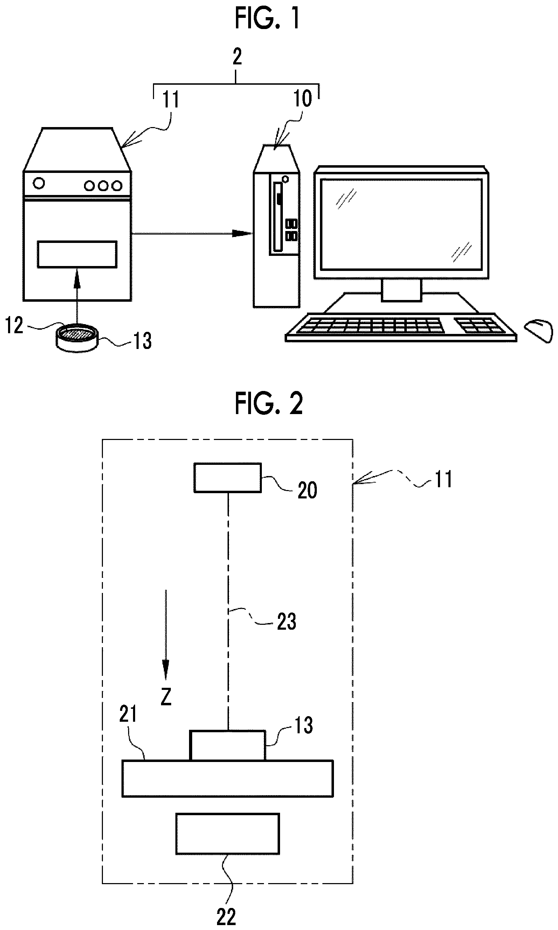

In Fig. 1, a digital holography system 2 is configured with an information processing apparatus 10 and a measurement apparatus 11. The information processing apparatus 10 is, for example, a desktop personal computer. The measurement apparatus 11 is connected to the information processing apparatus 10. A culture vessel 13 of a cell 12 is introduced into the measurement apparatus 11. The cell 12 is an example of an "object to be observed" according to the technique of the present disclosure. In the specification, the "cell" includes not only a single cell that is independently present, but also a cell aggregation where a plurality of cells are present as an aggregation.

In Fig. 2, the measurement apparatus 11 comprises a light source 20, a stage 21, and an imaging element 22. The light source 20 is, for example, a laser diode (LD). Alternatively, the light source 20 is a combination of a light emitting diode (LED) and a pinhole. The light source 20 emits coherent light 23 toward the culture vessel 13 placed on the stage 21. The coherent light 23 is incident on the cell 12 and the culture vessel 13. The coherent light 23 is an example of "illumination light" according to the technique of the present disclosure. A Z direction indicated by an arrow is an irradiation direction of the coherent light 23.

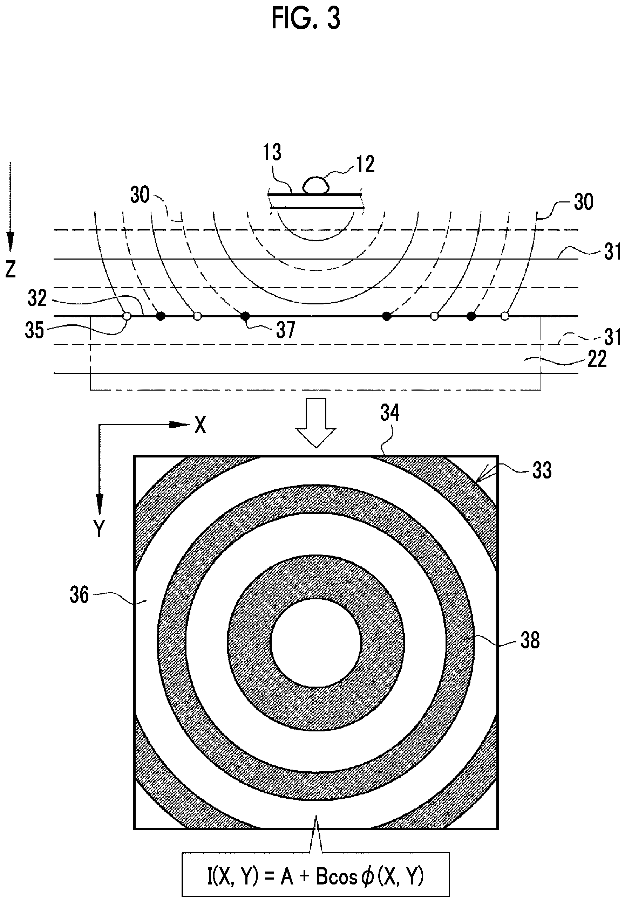

As shown in Fig. 3, the coherent light 23 incident on the cell 12 and the culture vessel 13 is divided into object light 30 diffracted by the cell 12 and the culture vessel 13 and transmitted light 31 transmitted without passing through the cell 12 and the culture vessel 13. The object light 30 and the transmitted light 31 interfere on an imaging surface 32 of the imaging element 22, and produce interference fringes 33. The imaging element 22 images the interference fringes 33 and outputs an interference fringe image 34. The transmitted light 31 is an example of "reference light" according to the technique of the present disclosure.

A pixel value of each pixel of the interference fringe image 34 is intensity I of the interference fringes 33. That is, the interference fringe image 34 is a two-dimensional distribution of the intensity I of the interference fringes 33. The intensity I of the interference fringes 33 is represented by Expression (1) described below where a phase difference between the object light 30 and the transmitted light 31 is φ.

$$I(X,Y) = A + B\cos\varphi(X,Y) \ ... \ (1)$$

A and B are constants. (X,Y) is an X coordinate and a Y coordinate of each pixel of the interference fringe image 34.

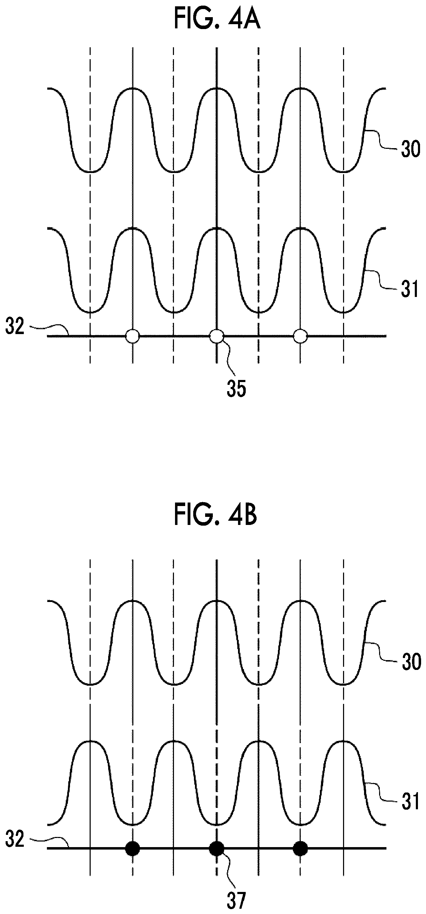

As also shown in Figs. 4A and 4B, among lines representing the object light 30 and the transmitted light 31, solid lines indicate wave fronts with maximum amplitude of the object light 30 and the transmitted light 31. In contrast, broken lines indicate wave fronts with minimum amplitude of the object light 30 and the transmitted light 31. White points 35 shown on the imaging surface 32 are portions where the wave fronts of the object light 30 and the transmitted light 31 are equalized and intensified (see Fig. 4A). The portions of the white points 35 appear as bright portions 36 in the interference fringes 33. In contrast, black points 37 shown on the imaging surface 32 are portions where the wave fronts of the object light 30 and the transmitted light 31 are shifted by a half wavelength and weakened (see Fig. 4B). The portions of the black points 37 appear as dark portions 38 in the interference fringes 33.

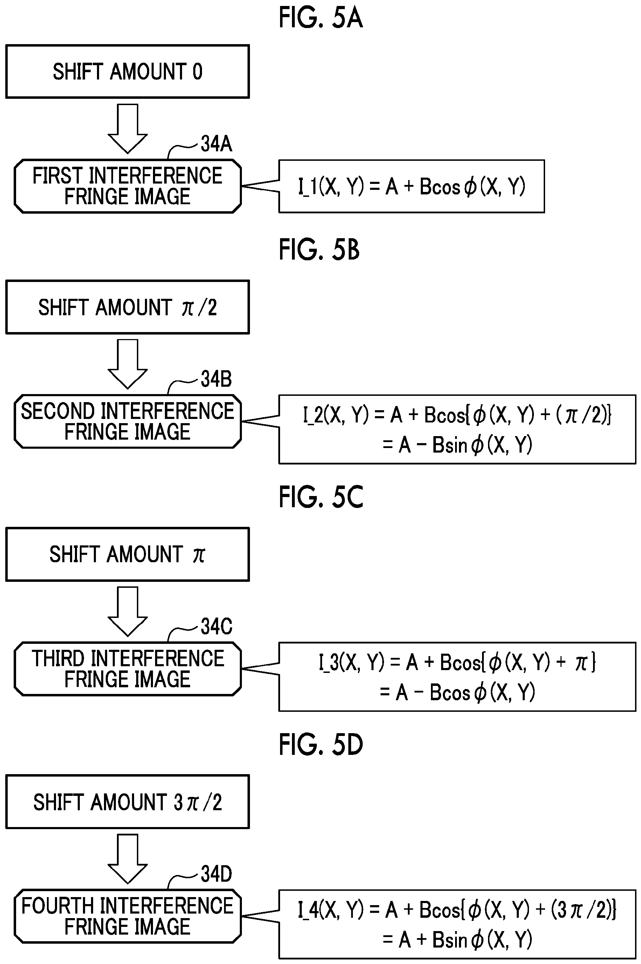

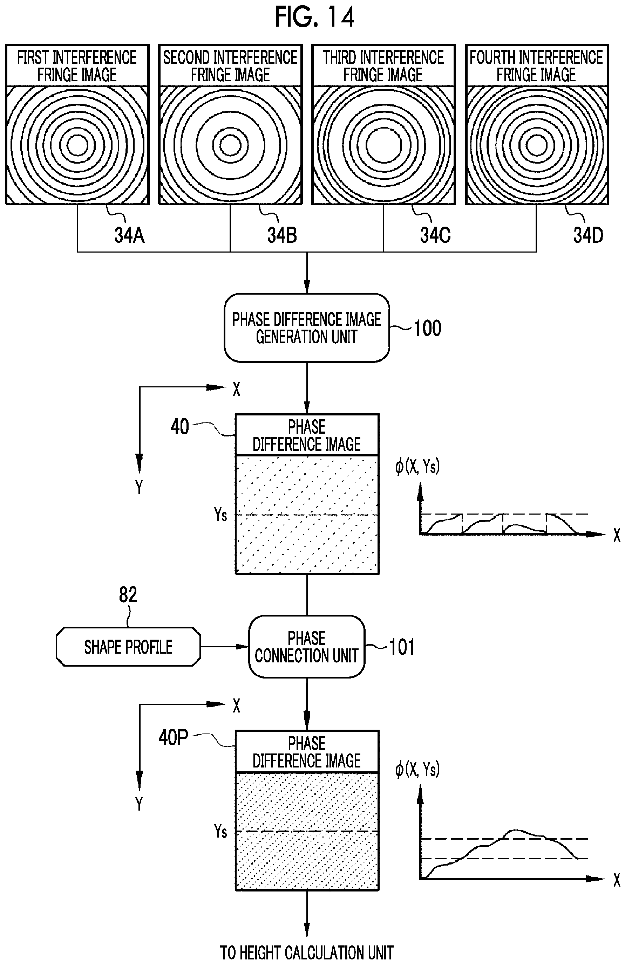

As shown in Figs. 5A to 5D, in the measurement apparatus 11, an optical path difference between the object light 30 and the transmitted light 31 is shifted by $\pi/2$, and the interference fringe image 34 is output from the imaging element 22 each time. Specifically, irradiation of the coherent light 23 by the light source 20 and imaging of the interference fringe image 34 by the imaging element 22 are performed four times in total of a shift amount of 0 shown in Fig. 5A, a shift amount of $\pi/2$ shown in Fig. 5B, a shift amount of $\pi$ shown in Fig. 5C, and a shift amount of $3\pi/2$ shown in Fig. 5D. With this, a first interference fringe image 34A in a case of the shift amount of 0 shown in Fig. 5A, a second interference fringe image 34B in a case of the shift amount of $\pi/2$ shown in Fig. 5B, a third interference fringe image 34C in a case of the shift amount of $\pi$ shown in Fig. 5C, and a fourth interference fringe image 34D in a case of the shift amount of $3\pi/2$ shown in Fig. 5D are obtained. In the first interference fringe image 34A, the second interference fringe image 34B, the third interference fringe image 34C, and the fourth interference fringe image 34D, since the optical path difference between the object light 30 and the transmitted light 31 is different, the bright portions 36 and the dark portions 38 of the interference fringes 33 are different in width even with the same cell 12 and the culture vessel 13 (see Fig. 14).

In this way, a method of outputting the interference fringe image 34 while shifting the optical path difference between the object light 30 and the transmitted light 31 by a specified amount is referred to as a phase shift method. In particular, a method of setting the specified amount to $\pi/2$ and the four interference fringe images 34 in total as described above is referred to as a four-step method. In the following description, unless there is no need for particular distinction, the first interference fringe image 34A, the second interference fringe image 34B, the third interference fringe image 34C, and the fourth interference fringe image 34D are collectively referred to as the interference fringe images 34.

Intensity of I_1(X,Y) of the interference fringes 33 shown in the first interference fringe image 34A is represented by Expression (2) described below.

$$I\_1(X,Y) = A + B\cos\varphi(X,Y) \ldots (2)$$

Similarly, intensity I 2(X,Y) of the interference fringes 33 shown in the second interference fringe image 34B, intensity I_3(X,Y) of the interference fringes 33 shown in the third interference fringe image 34C, and intensity I 4(X,Y) of the interference fringes 33 shown in the fourth interference fringe image 34D are represented by Expressions (3), (4), and (5) described below.

$$I\_2(X,Y) = A + B\cos\{\varphi(X,Y) + (\pi/2)\} \ldots (3)$$

$$I\_3(X,Y) = A + B\cos\{\varphi(X,Y) + \pi\} \ldots (4)$$

$$I\_4(X,Y) = A + B\cos\{\varphi(X,Y) + (3\pi/2)\} \ldots (5)$$

Expressions (3), (4), and (5) are rewritten to Expressions (3A), (4A), and (5A) described below.

$$I\_2(X,Y) = A - B\sin\varphi(X,Y) \ldots (3A)$$

$$I\_3(X,Y) = A - B\cos\varphi(X,Y) \ldots (4A)$$

$$I\_4(X,Y) = A + B\sin\varphi(X,Y) \ldots (5A)$$

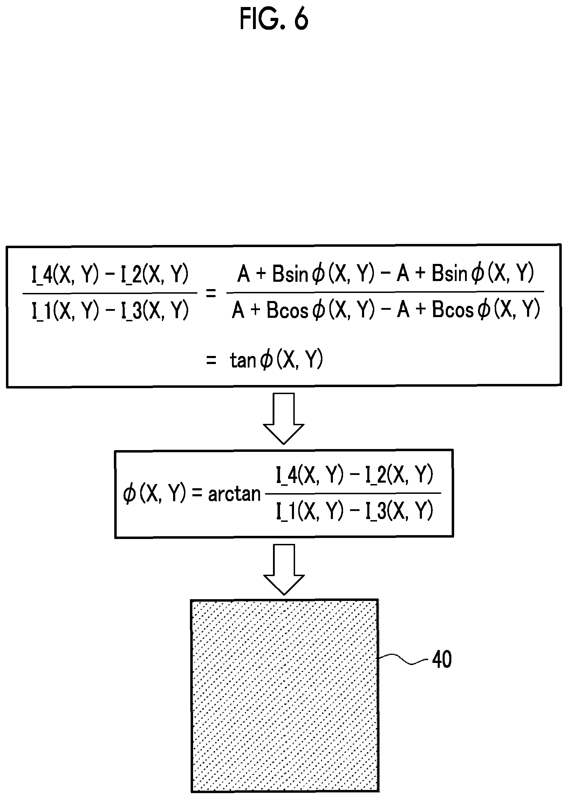

In Fig. 6, Expression (6) described below is derived from Expressions (2), (3A), (4A), and (5A).

$$\{I\_4(X,Y) - I\_2(X,Y)\}/\{I\_1(X,Y) - I\_3(X,Y)\} = \tan\varphi(X,Y) \ldots (6)$$

Accordingly, a phase difference $\varphi(X,Y)$ between the object light 30 and the transmitted light 31 is represented by Expression (7) described below.

$$\varphi(X,Y) = \arctan\{I\_4(X,Y) - I\_2(X,Y)\}/\{I\_1(X,Y) - I\_3(X,Y)\} \ldots (7)$$

That is, the phase difference $\varphi(X,Y)$ can be obtained from simple computation using the intensity $I\_1(X,Y)$ to $I4(X,Y)$ of the interference fringes 33 of the first interference fringe image 34A to the fourth interference fringe image 34D. The phase difference $\varphi(X,Y)$ obtained in this manner is set as a pixel value of a pixel corresponding to the pixel of the interference fringe image 34, whereby a phase difference image 40 that is a two-dimensional distribution of the phase difference $\varphi$ is obtained.

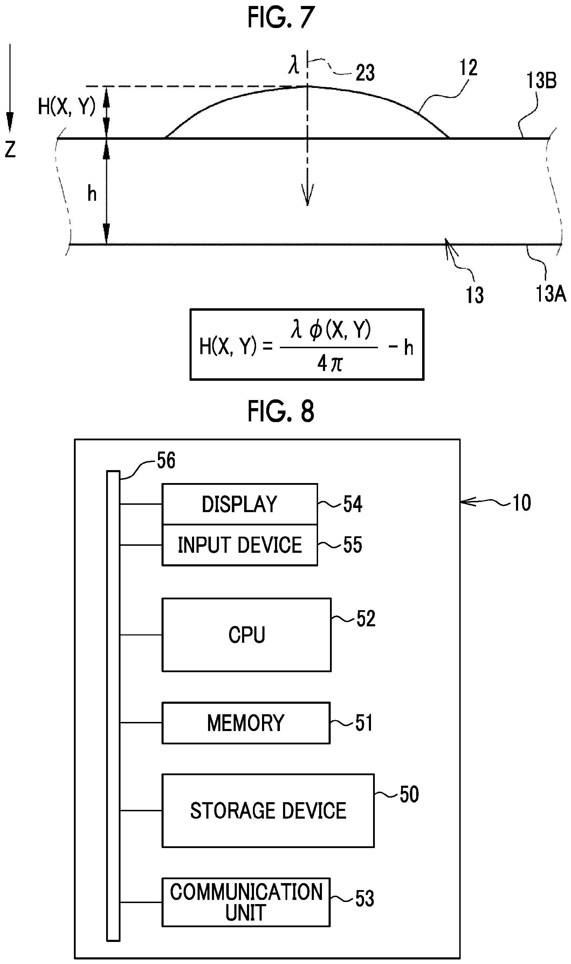

As shown in Fig. 7, a height $H(X,Y)$ of the cell 12 along the irradiation direction Z of the coherent light 23 is represented by Expression (8) described below.

$$H(X,Y) = \{\lambda\varphi(X,Y)/4\pi\} - h \ldots (8)$$

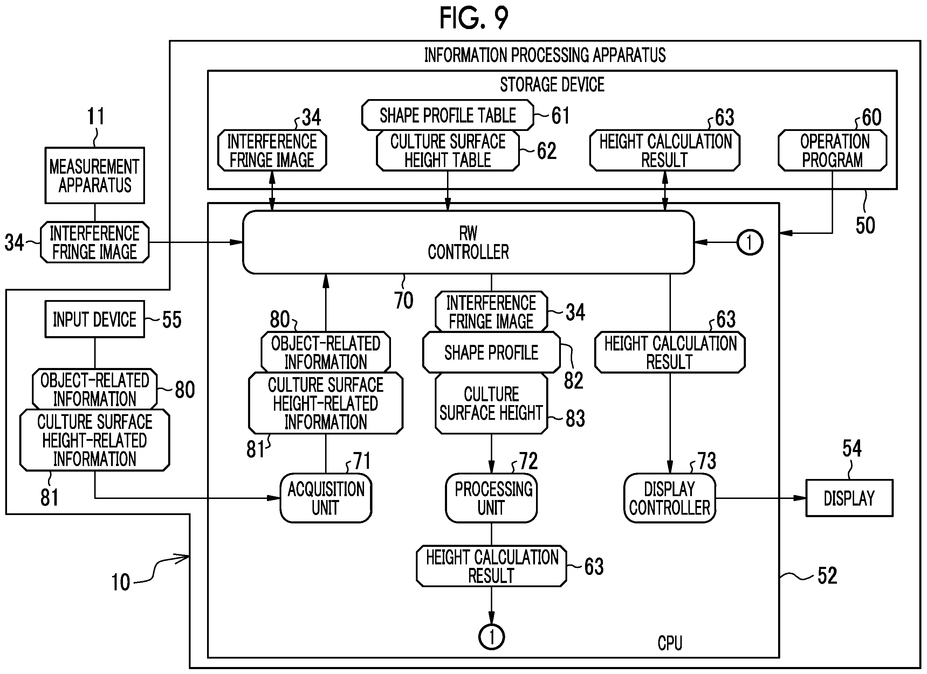

$\lambda$ is a wavelength of the coherent light 23, and is, for example, 640 nm. h is a height (hereinafter, simply referred to as a culture surface height) 83 (see Fig. 9) from a bottom surface 13A of the culture vessel 13 placed on the stage 21 and a culture surface 13B of the culture vessel 13 where the cell 12 is cultured. In this way, in a case where the phase difference $\varphi(X,Y)$ or the like is known, the height $H(X,Y)$ of the cell 12 can be obtained. The height $H(X,Y)$ of the cell 12 is an example of "a shape of an object to be observed" according to the technique of the present disclosure.

In Fig. 8, a computer that configures the information processing apparatus 10 comprises a storage device 50, a memory 51, a central processing unit (CPU) 52, a communication unit 53, a display 54, and an input device 55. These are connected through a bus line 56.

The storage device 50 is an example of a "storage unit" according to the technique of the present disclosure. The storage device 50 is a hard disk drive incorporated in the computer that configures the information processing apparatus 10 or connected to the computer through a cable or a network. Alternatively, the storage device 50 is a disk array where a plurality of hard disk drives are connected. In the storage device 50, a control program, such as an operating system, various application programs, various kinds of data accompanied with such programs, and the like are stored. A solid state drive may be used instead of the hard disk drive.

The memory 51 is a work memory on which the CPU 52 executes processing. The CPU 52 loads the programs stored in the storage device 50 to the memory 51 and executes processing depending on the programs, thereby integrally controlling each unit of the computer. The CPU 52 is an example of a "processor" according to the technique of the present disclosure.

The communication unit 53 is a network interface that performs transmission control of various kinds of information through a network, such as a local area network (LAN). The display 54 displays various screens. The computer that configures the information processing apparatus 10 receives an input an operation instruction from the input device 55 through various screens. The input device 55 is a keyboard, a mouse, a touch panel, and the like.

In Fig. 9, the storage device 50 of the information processing apparatus 10 stores an operation program 60. The operation program 60 is an application program that causes the computer to function as the information processing apparatus 10. That is, the operation program 60 is an example of "an operation program for an information processing apparatus" according to the technique of the present disclosure. In the storage device 50, the interference fringe image 34, a shape profile table 61, a culture surface height table 62, and a calculation result (hereinafter, referred to as a height calculation result) 63 of the height $H(X,Y)$ of the cell 12 are also stored.

In a case where the operation program 60 is started, the CPU 52 of the computer that configures the information processing apparatus 10 functions as a read write (hereinafter, abbreviated as RW) controller 70, an acquisition unit 71, a processing unit 72, and a display controller 73 in cooperation with the memory 51 and the like.

The RW controller 70 controls storage of various kinds of data in the storage device 50 and readout of various kinds of data in the storage device 50. For example, the RW controller 70 receives the interference fringe image 34 from the measurement apparatus 11 and stores the interference fringe image 34 in the storage device 50. The RW controller 70 reads out the interference fringe image 34 from the storage device 50 and outputs the interference fringe image 34 to the processing unit 72.

The acquisition unit 71 acquires object-related information 80 and culture surface height-related information 81 that are input from a user through the input device 55. The object-related information 80 is information regarding the cell 12 that is an object to be observed. The culture surface height-related information 81 is information regarding the culture surface height 83. The acquisition unit 71 outputs the object-related information 80 and the culture surface height-related information 81 to the RW controller 70.

The RW controller 70 reads out a shape profile 82 corresponding to the object-related information 80 from the acquisition unit 71, from the shape profile table 61 of the storage device 50. That is, the RW controller 70 is an example of a "readout unit" according to the technique of the present disclosure. The RW controller 70 outputs the shape profile 82 to the processing unit 72.

The RW controller 70 reads out the culture surface height 83 corresponding to the culture surface height-related information 81 from the acquisition unit 71, from the culture surface height table 62 of the storage device 50. The RW controller 70 outputs the culture surface height 83 to the processing unit 72.

The processing unit 72 calculates the height H(X,Y) of the cell 12 based on the interference fringe image 34, the shape profile 82, and the culture surface height 83. The processing unit 72 outputs the height calculation result 63 to the RW controller 70.

The RW controller 70 stores the height calculation result 63 from the processing unit 72 in the storage device 50. The RW controller 70 reads out the height calculation result 63 from the storage device 50 and outputs the height calculation result 63 to the display controller 73.

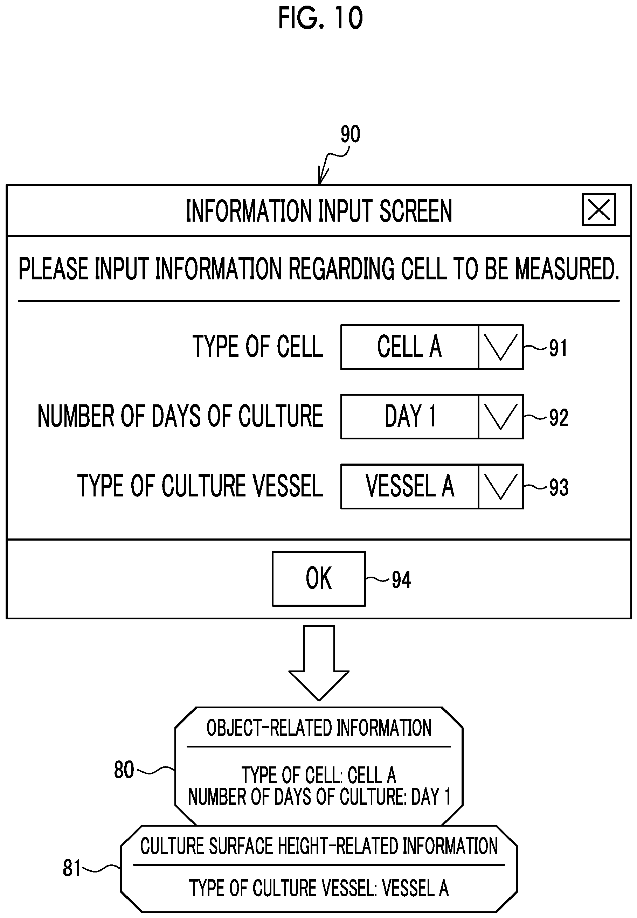

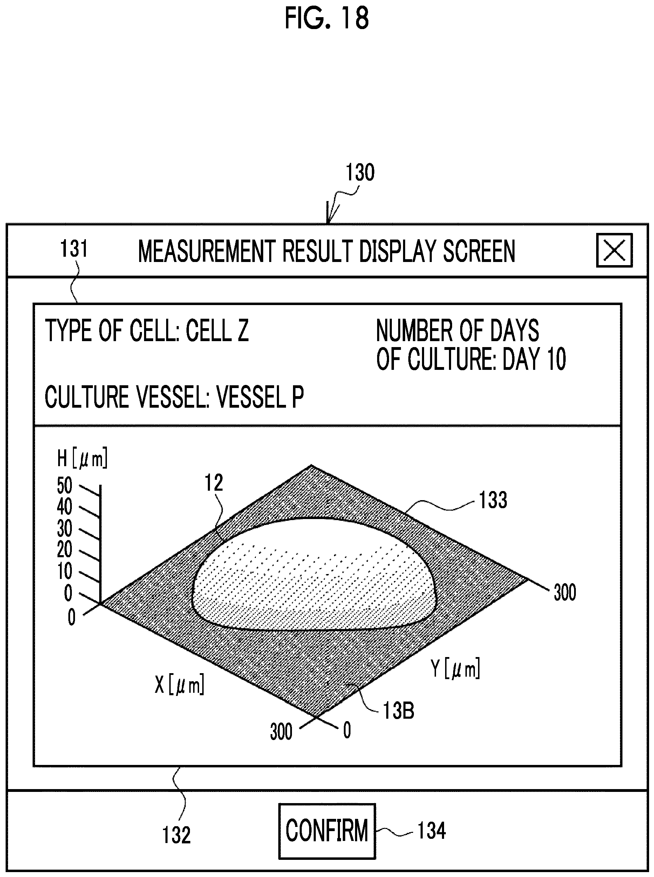

The display controller 73 controls the display of various screens on the display 54. Various screens include an information input screen 90 (see Fig. 10) on which the object-related information 80 and the culture surface height-related information 81 are input, a measurement result display screen 130 (see Fig. 18) on which the height calculation result 63 is displayed, and the like.

As shown in Fig. 10, the information input screen 90 is provided with three pull-down menus 91, 92, and 93. The pull-down menu 91 is a graphical user interface (GUI) for selecting and inputting a type of the cell 12 as the object-related information 80. The pull-down menu 92 is a GUI for selecting and inputting the number of days of culture as the object-related information 80. The pull-down menu 93 is a GUI for selecting and inputting a type of the culture vessel 13 as the culture surface height-related information 81. The number of days of culture is an example of a "culture condition" according to the technique of the present disclosure.

The user operates the pull-down menus 91 to 93 to select desired options and selects an OK button 94. With this, the object-related information 80 and the culture surface height-related information 81 are acquired in the acquisition unit 71. In Fig. 10, a case where a cell A is selected and input in the type of the cell 12, day 1 is selected and input in the number of days of culture, and vessel A is selected and input in the type of the culture vessel 13 is shown.

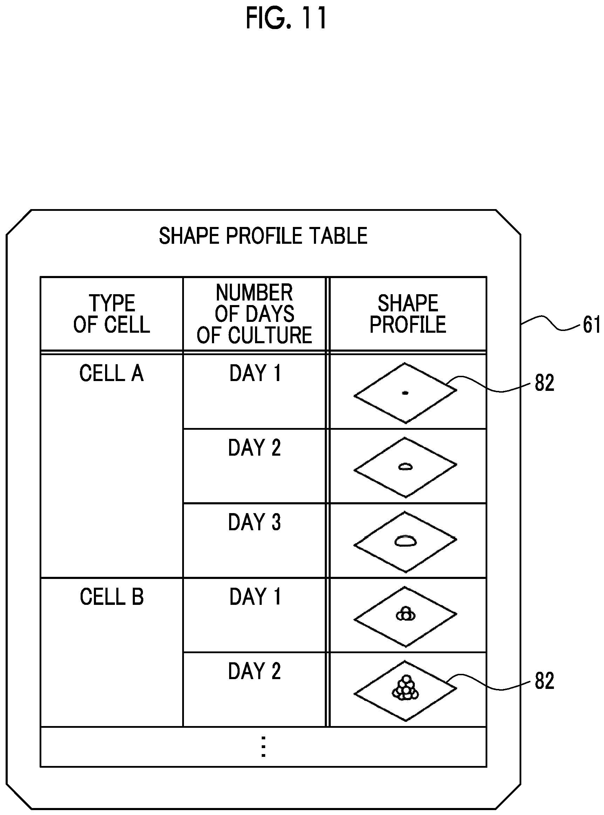

In Fig. 11, in the shape profile table 61, the shape profile 82 is registered for each type of the cell 12 and the number of days of culture. That is, in the shape profile table 61, the object-related information 80 and the shape profile 82 are stored in association with each other. The shape profile 82 is data indicating a typical shape of the cell 12 that is assumed in a case where the cell 12 corresponding to the number of days of culture and the type is cultured. In more detail, the shape profile 82 is data indicating a three-dimensional size including the height of the cell 12. For example, in the shape profile 82, numerical values regarding the three-dimensional size of the cell 12, such as a maximum height of the cell 12, widths in an X direction and a Y direction of the cell 12, and in a case were the cell 12 has a recess, a depth and a width of the recess, are included.

The shape of the cell 12 is different depending on the type of cell 12. For example, in a case of a red blood cell of a human, a central portion is recessed by several $\mu$m, and in a case of a mesenchymal stem cell, a region of a nucleus is thick, about 10 $\mu$m, and a region of cytoplasm is thin, about several $\mu$m. In a case where the culture condition, such as the number of days of culture, is different, the shape of the cell 12 is also naturally different. The shape profile 82 is data representing such a feature of the shape of the cell 12.

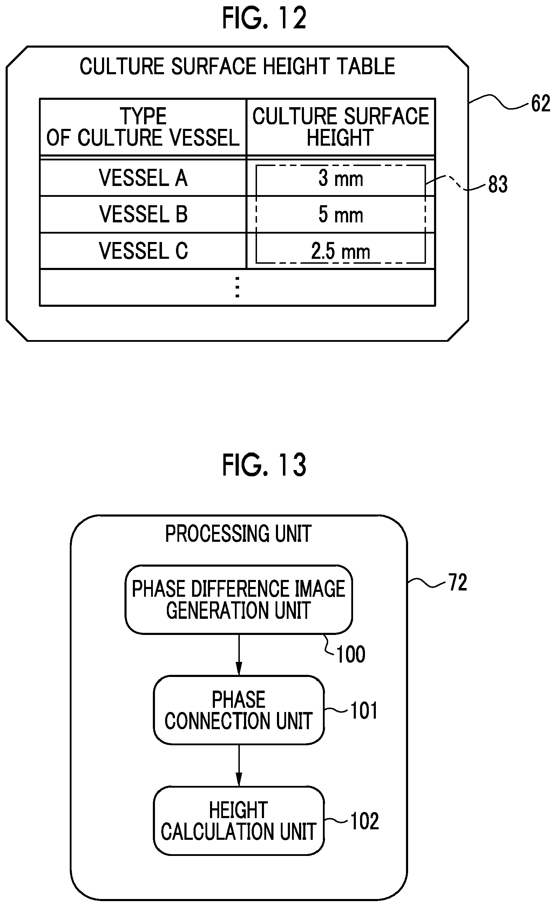

In Fig. 12, in the culture surface height table 62, the culture surface height 83 is registered for each type of the culture vessel 13.

In Fig. 13, the processing unit 72 has a phase difference image generation unit 100, a phase connection unit 101, and a height calculation unit 102. The phase difference image generation unit 100 generates the phase difference image 40. The phase connection unit 101 performs phase connection with respect to the phase difference image 40. The height calculation unit 102 calculates the height H(X,Y) of the cell 12 based on a phase difference image 40P (see Fig. 14) after the phase connection.

As shown in Fig. 14, the first interference fringe image 34A to the fourth interference fringe image 34D are input to the phase difference image generation unit 100. The phase difference image generation unit 100 generates the phase difference image 40 from the first interference fringe image 34A to the fourth interference fringe image 34D as shown in Fig. 6. The phase difference image generation unit 100 outputs the phase difference image 40 to the phase connection unit 101.

The phase difference $\varphi$(X,Y) that is the pixel value of the phase difference image 40 is a function of arctan as shown in Expression (7). For this reason, as shown in a graph of a phase difference $\varphi$(X,Ys) in a certain row Ys of the phase difference image 40, the phase difference $\varphi$(X,Y) is obtained to be folded in a range of $-\pi$ to $\pi$, and a phase jump of about $\pm 2\pi$ occurs in places. The phase connection unit 101 performs the phase connection for connecting the phase difference $\varphi$(X,Y) where there is such a phase jump, with respect to the phase difference image 40. In this case, the phase connection unit 101 refers to the shape profile 82. The phase connection unit 101 outputs the phase difference image 40P after the phase connection to the height calculation unit 102.

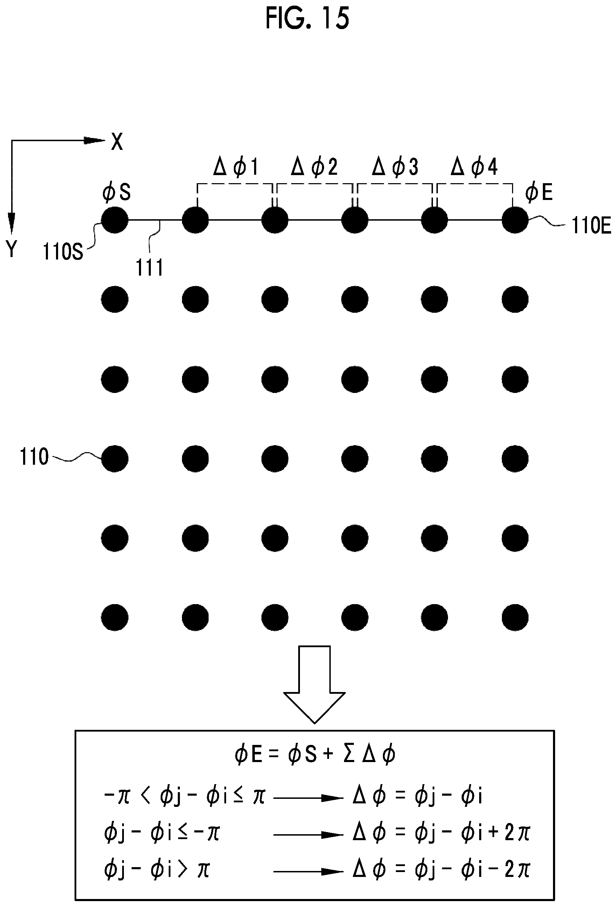

As shown in Fig. 15, the phase connection unit 101 performs the phase connection in the following manner in principle. In Fig. 15, a case of obtaining a phase difference $\varphi$E of a head pixel 110E following a path 111 along the X direction with a phase difference $\varphi$S that is a pixel value of a pixel 110S where the phase connection starts, as a reference in each pixel 110 of the phase difference image 40 will be described as an example. In this case, the phase difference $\varphi$E of the pixel 110E is obtained by adding a difference $\Delta\varphi$ of the phase difference $\varphi$S of the pixel 110S and a total of a difference between phase differences φ of two adjacent pixels 110 connected by the path 111 other than the pixel 110S. That is, the phase difference φE of the pixel 110E is represented by Expression (9) described below.

$$\varphi E = \varphi S + \Sigma \Delta \varphi \ ... \ (9)$$

Note that, in a case where the phase difference of the pixel 110 on the pixel 110S side among the two adjacent pixels 110 connected by the path 111 is φi, and the phase difference of the pixel 110 on the pixel 110E side is cpj, Δφ changes depending on the value of cpj - φi as Expressions (10), (11), and (12). That is,

$$\text{In a case where } -\pi < \varphi j - \varphi i \leq \pi, \Delta \varphi = \varphi j - \varphi i \ ... \ (10)$$

$$\text{In a case where } \varphi j - \varphi i \leq -\pi, \Delta \varphi = \varphi j - \varphi i + 2\pi \ ... \ (11)$$

$$\text{In a case where } \varphi j - \varphi i > \pi, \Delta \varphi = \varphi j - \varphi i - 2\pi \ ... \ (12)$$

In the case of Expression (10), a phase jump does not occur in the two adjacent pixels 110 connected by the path 111. For this reason, cpj - φi is employed as Δφ as it is. In contrast, in the cases of Expressions (11) and (12), a phase jump occurs in the two adjacent pixels 110 connected by the path 111. For this reason, $2\pi$ is added to or subtracted from cpj - φi.

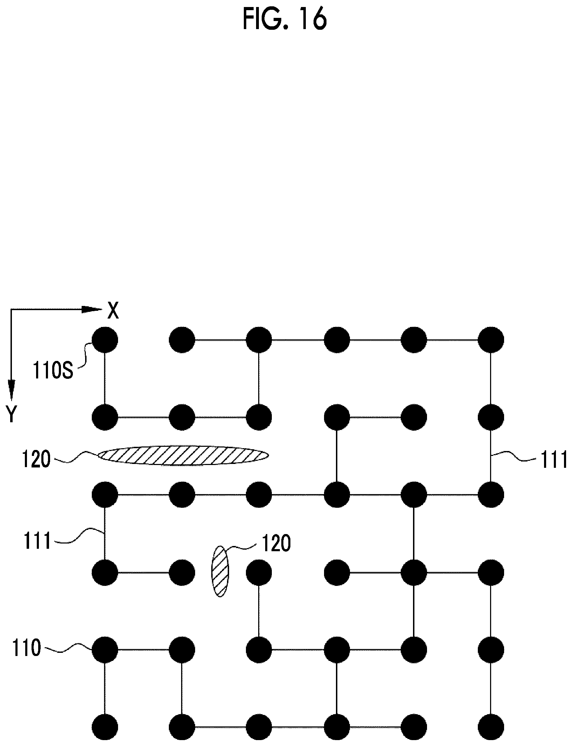

In this way, the phase connection is processing of successively adding the difference Δφ between the phase differences φ of the two adjacent pixels 110 connected by the path 111. For this reason, miscalculation of Δφ in the middle of the path 111 influences the calculation result of the phase difference φE. Accordingly, actually, the phase connection unit 101 selects the path 111 while avoiding a place 120 where Δφ is likely to be miscalculated as shown in Fig. 16, instead of the linear path 111 shown in Fig. 15. As a method of selecting such an optimum path of the phase connection, for example, a minimum spanning tree (MST) method is used.

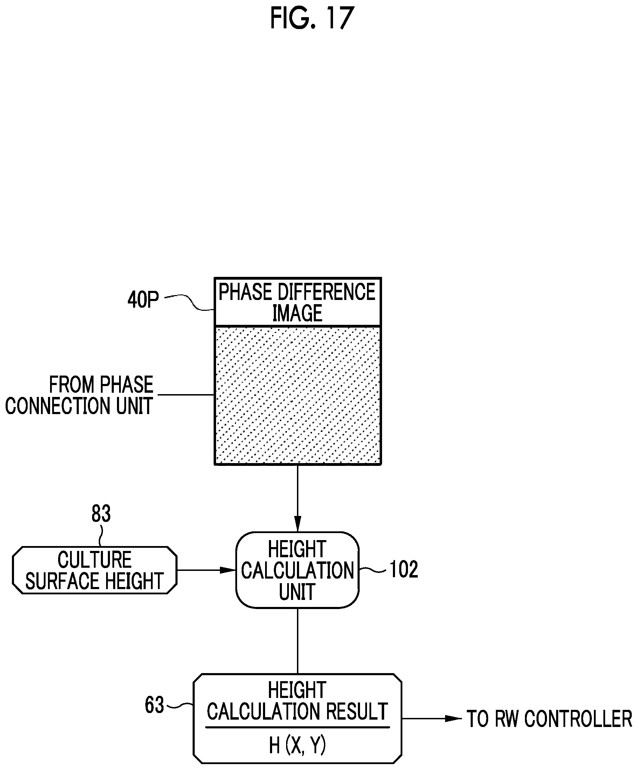

In Fig. 17, the height calculation unit 102 calculates the height H(X,Y) of the cell 12 based on phase difference image 40P after the phase connection from the phase connection unit 101 and the culture surface height 83. The height calculation unit 102 outputs the height calculation result 63 to the RW controller 70.

As shown in Fig. 18, the measurement result display screen 130 is provided with an information display region 131 and a measurement result display region 132. In the information display region 131, the type of the cell 12, the number of days of culture, and the type of the culture vessel 13 input through the information input screen 90 are displayed. In the measurement result display region 132, a three-dimensional color map 133 of the height H(X,Y) of the cell 12 is displayed. The three-dimensional color map 133 represents the shape of the cell 12 in a three-dimensional manner, and colors places at the same height H(X,Y) with the same color. A zero point of the height H(X,Y) of the three-dimensional color map 133 is the culture surface 13B of the culture vessel 13. The display of the measurement result display screen 130 is turned off in a case where a confirm button 134 is selected.

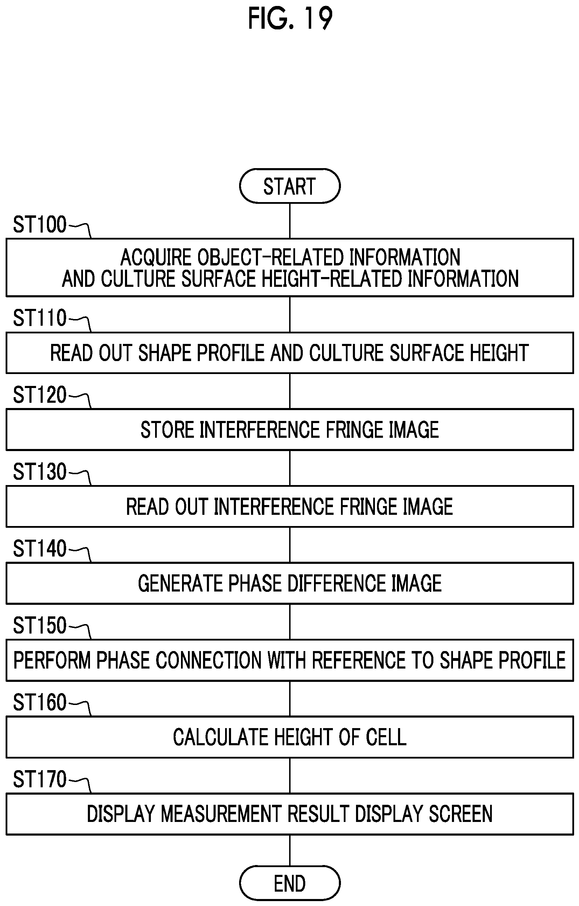

Next, the operations of the above-described configuration will be described referring to a flowchart of Fig. 19. In a case where the operation program 60 is started in the information processing apparatus 10, as shown in Fig. 9, the CPU 52 of the information processing apparatus 10 functions as the RW controller 70, the acquisition unit 71, the processing unit 72, and the display controller 73.

First, the object-related information 80 and the culture surface height-related information 81 input from the user through the information input screen 90 shown in Fig. 10 are acquired in the acquisition unit 71 (Step ST100). In the example, the object-related information 80 is the type of the cell 12 and the number of days of culture. The culture surface height-related information 81 is the type of the culture vessel 13. The object-related information 80 and the culture surface height-related information 81 are output from the acquisition unit 71 to the RW controller 70. Step ST100 is an example of an "acquisition step" according to the technique of the present disclosure.

The RW controller 70 reads out the shape profile 82 corresponding to the object-related information 80 from the acquisition unit 71, from the shape profile table 61 of the storage device 50. Similarly, the RW controller 70 reads out the culture surface height 83 corresponding to the culture surface height-related information 81 from the acquisition unit 71, from the culture surface height table 62 of the storage device 50 (Step ST110). The shape profile 82 and the culture surface height 83 are output from the RW controller 70 to the processing unit 72. Step ST110 is an example of a "readout step" according to the technique of the present disclosure.

In the measurement apparatus 11, as shown in Fig. 5, the optical path difference between the object light 30 and the transmitted light 31 is shifted by $\pi/2$, and the first interference fringe image 34A to the fourth interference fringe image 34D are output from the imaging element 22 each time. The first interference fringe image 34A to the fourth interference fringe image 34D are output from the measurement apparatus 11 to the information processing apparatus 10, and the RW controller 70 stores the first interference fringe image 34A to the fourth interference fringe image 34D in the storage device 50 (Step ST120).

The RW controller 70 reads out the first interference fringe image 34A to the fourth interference fringe image 34D from the storage device 50 and outputs the first interference fringe image 34A to the fourth interference fringe image 34D to the processing unit 72 (Step ST130). In the processing unit 72, as shown in Fig. 14, the phase difference image generation unit 100 generates the phase difference image 40 from the first interference fringe image 34A to the fourth interference fringe image 34D (Step ST140). The phase difference image 40 is output from the phase difference image generation unit 100 to the phase connection unit 101.

As shown in Figs. 14 to 16, the phase connection unit 101 performs the phase connection with respect to the phase difference image 40 while referring to the shape profile 82 (Step ST150). The phase difference image 40P after the phase connection is output from the phase connection unit 101 to the height calculation unit 102. Step ST150 is an example of a "phase connection step" according to the technique of the present disclosure.

As shown in Fig. 17, the height calculation unit 102 calculates the height H(X,Y) of the cell 12 based on the phase difference image 40P and the culture surface height 83 (Step ST160). The height calculation result 63 is output from the height calculation unit 102 to the RW controller 70, and the RW controller 70 stores the height calculation result 63 in the storage device 50.

The RW controller 70 reads out the height calculation result 63 from the storage device 50 and outputs the height calculation result 63 to the display controller 73. Then, the display controller 73 displays the measurement result display screen 130 shown in Fig. 18 on the display 54, and the three-dimensional color map 133 indicating the height calculation result 63 is provided for viewing by the user (Step ST170).

As described above, the acquisition unit 71 of the CPU 52 of the information processing apparatus 10 acquires the object-related information 80 regarding the cell 12 that is an object to be observed. The RW controller 70 reads out the shape profile 82 corresponding to the acquired object-related information 80 from the shape profile table 61 of the storage device 50. The phase connection unit 101 of the processing unit 72 performs the phase connection with respect to the phase difference image 40 with reference to the read-out shape profile 82 before obtaining the height H(X,Y) of the cell 12. For this reason, unlike JP2006-284186A of the related art, it is possible to reduce a processing time of the phase connection without needing a special mechanism for measuring a rough shape of the cell 12 and without spending an excess measurement time.

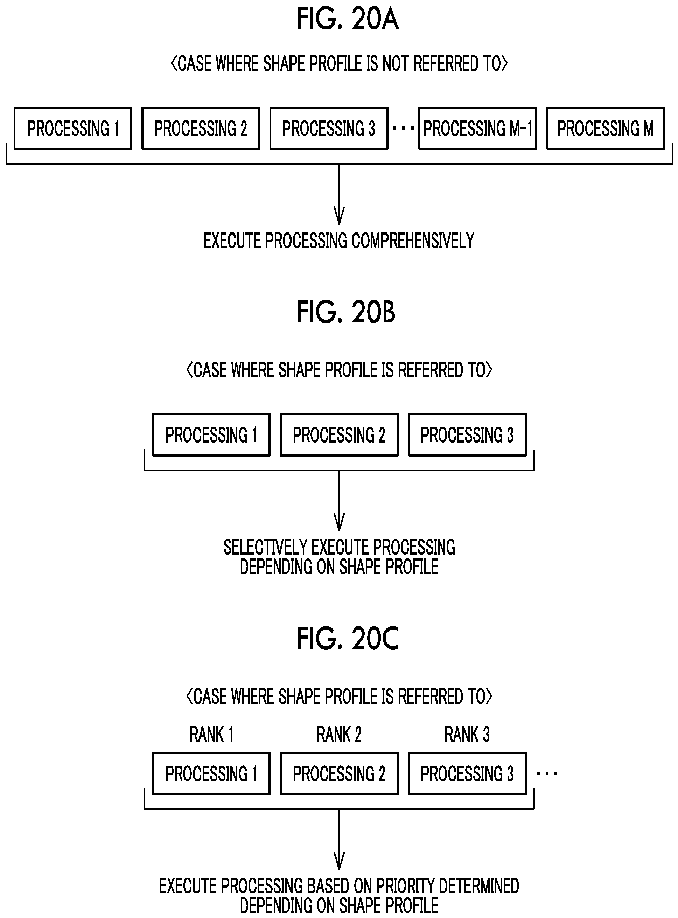

Figs. 20A to 20C are diagrams illustrating the effects of the technique of the present disclosure that it is possible to reduce the processing time of the phase connection. First, as shown in Fig. 20A, in a case where the shape profile 82 is not referred to, the phase connection unit 101 should execute processing 1 to processing M regarding the calculation of $\Delta\varphi$ and the selection of the path 111 comprehensively without any guideline. In contrast, as shown in Fig. 20B, in the technique of the present disclosure, as the shape profile 82 is referred to, the phase connection unit 101 just executes a part of processing among the processing 1 to the processing M regarding the calculation of $\Delta\varphi$ and the selection of the path 111, for example, the processing 1 to the processing 3. Alternatively, as shown in Fig. 20C, the phase connection unit 101 decides the priority of the processing depending on the shape profile 82. Then, the processing is executed following the priority, and at the time at which processing of higher ranks, for example, the processing 1 to the processing 3 of the rank 1 to the rank 3 are executed, the phase connection reaches a satisfying result. Thus, the phase connection ends. For this reason, it is possible to reduce the processing time of the phase connection.

The CPU 52 performs control for displaying the height calculation result 63 of the cell 12. For this reason, it is possible to notify the user of the height calculation result 63.

A field of cell culture is recently highlighted due to the appearance of an induced pluripotent stem (iPS) cell or the like. For this reason, there is a demand for a technique for analyzing the cell 12 during culture in detail. In the technique of the present disclosure, an object to be observed is the cell 12 during culture. Accordingly, it can be said that the technique of the present disclosure is a technique capable of meeting a recent demand.

The CPU 52 acquires the culture surface height-related information 81 regarding the culture surface height 83. For this reason, it is possible to determine the reference of the height H(X,Y) of the cell 12 to the culture surface 13B, and to more accurately calculate the height H(X,Y) of the cell 12.

The object-related information 80 is the type of the cell 12 and the culture condition of the cell. For this reason, it is possible to obtain the shape profile 82 appropriate for the type of the cell 12 and the culture condition of the cell.

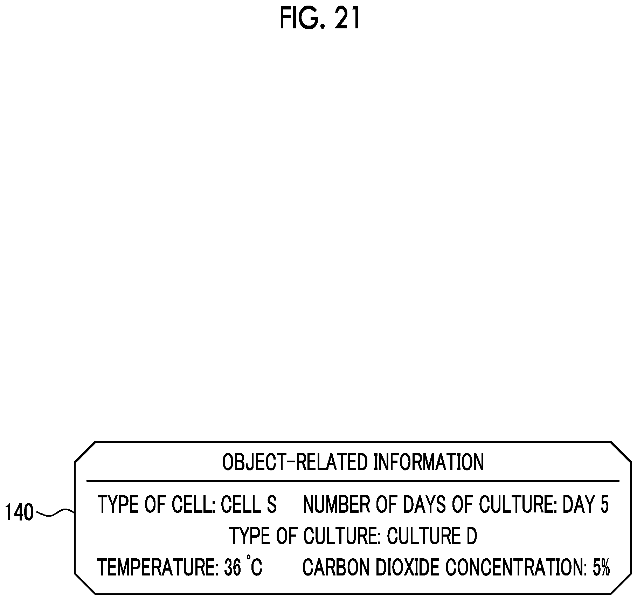

The culture condition of the cell included in the object-related information is not limited to the number of days of culture illustrated. Like object-related information 140 shown in Fig. 21, a type of a culture medium, a temperature of a culture environment, and a carbon dioxide concentration of the culture environment may be included. Since the culture medium provides nutrients necessary for the growth of the cell 12, the type of the culture medium is an important parameter that influences the growth of the cell 12. Since the temperature of the culture environment and the carbon dioxide concentration also promote or obstruct the growth of the cell 12 by values, the temperature of the culture environment and the carbon dioxide concentration are important parameters. Needless to say, the number of days of culture is also an important parameter regarding the shape of the cell 12. For this reason, in a case where the number of days of culture, the type of the culture medium, the temperature of the culture environment, and the carbon dioxide concentration of the culture environment are included in the culture condition, it is possible to obtain the more detailed and accurate shape profile 82. In this case, the type of the culture medium, the temperature of the culture environment, and the carbon dioxide concentration of the culture environment are added to the items of the shape profile table.

The culture condition of the cell included in the object-related information may include at least any one of the number of days of culture, the type of the culture medium, the temperature of the culture environment, or the carbon dioxide concentration of the culture environment illustrated above. In addition to these, a type of a culture solution, pH, osmotic pressure, an oxygen concentration of the culture environment, and the like may be further added.

The culture surface height-related information 81 may be a model number or the like of the culture vessel 13. The culture surface height-related information 81 may be the culture surface height 83 itself. In this case, the culture surface height table 62 is not required.

[Second Embodiment]

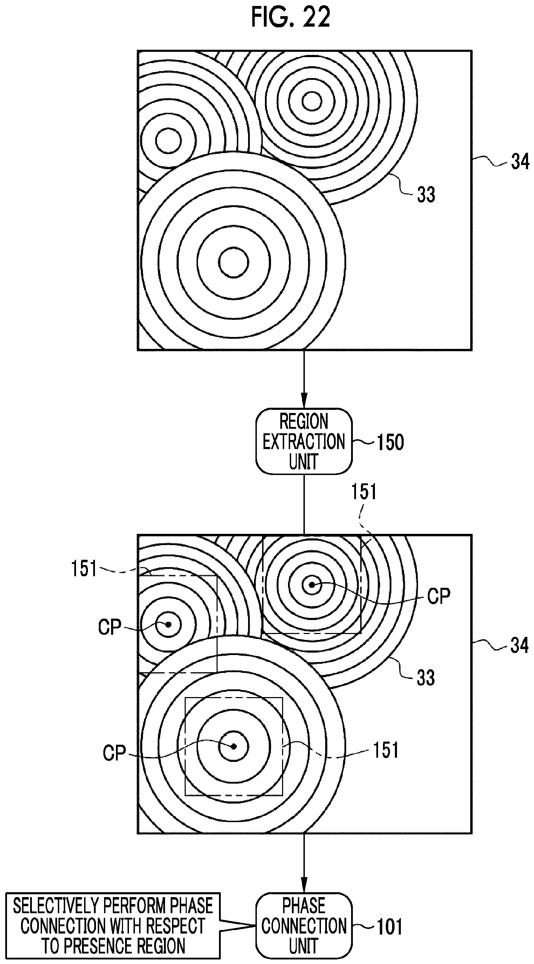

In the first embodiment described above, although an example where the phase connection is performed with respect to the entire phase difference image 40 has been shown, the technique of the present disclosure is not limited thereto. Like a second embodiment shown in Fig. 22, phase connection may be selectively performed with respect to a presence region 151 of the cell 12.

In Fig. 22, a CPU 52 of an information processing apparatus 10 of the second embodiment functions as a region extraction unit 150 in addition to the above-described units 70 to 73 and 100 to 102. The region extraction unit 150 extracts the presence region 151 of the cell 12 from the interference fringe image 34. The region extraction unit 150 extracts, for example, a square region of a predetermined size centering on a center CP of the interference fringes 33 shown in the interference fringe image 34, as the presence region 151 of the cell 12. The region extraction unit 150 outputs coordinate information of the presence region 151 to the phase connection unit 101. The phase connection unit 101 selectively performs the phase connection with respect to a region of the phase difference image 40 corresponding to the presence region 151.

In this way, in the second embodiment, the region extraction unit 150 extracts the presence region 151 of the cell 12 from the interference fringe image 34, and the phase connection unit 101 selectively performs the phase connection with respect to the presence region 151. For this reason, it is possible to further reduce the processing time of the phase connection.

[Third Embodiment]

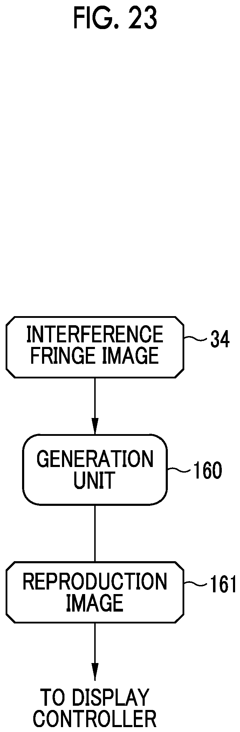

In the first embodiment described above, although the three-dimensional color map 133 has been illustrated as a display method of the height calculation result 63, the technique of the present disclosure is not limited thereto. Like a third embodiment shown in Figs. 23 to 25, the height calculation result 63 may be displayed on a reproduction image 161 representing any tomographic plane 166 of the cell 12 in a superimposed manner.

In Fig. 23, a CPU 52 of an information processing apparatus 10 of the third embodiment functions as a generation unit 160 in addition to the above-described units 70 to 73 and 100 to 102. The generation unit 160 generates the reproduction image 161 from the interference fringe image 34, for example, using known arithmetic processing, such as arithmetic processing by a Fourier iterative phase retrieval method. The generation unit 160 outputs the reproduction image 161 to the display controller 73.

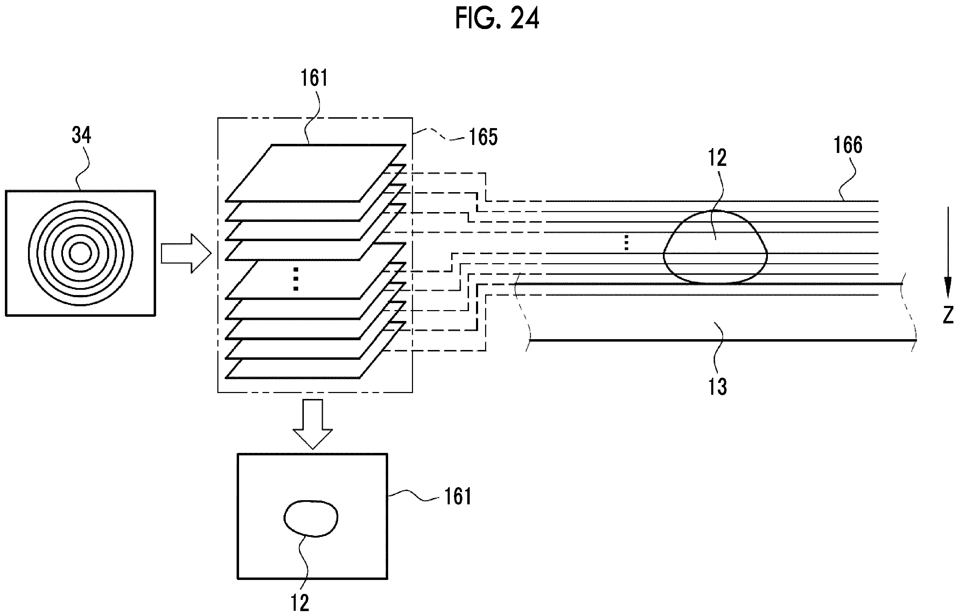

Fig. 24 shows the outline of arithmetic processing by the generation unit 160. The generation unit 160 first generates a reproduction image group 165 from the interference fringe image 34. The reproduction image group 165 is a group of a plurality of reproduction images 161. A plurality of reproduction images 161 are images representing tomographic planes 166 arranged at regular intervals in a height (thickness) direction of the cell 12 and the culture vessel 13 along the Z direction.

The generation unit 160 selects one best focused reproduction image 161 from among a plurality of reproduction images 161 of the reproduction image group 165. The generation unit 160 outputs the selected reproduction image 161 to the display controller 73. As a method of selecting the best focused reproduction image 161, a method of calculating a contrast value of each of a plurality of reproduction images 161 and selecting the reproduction image 161 having the highest contrast value as the best focused reproduction image 161, or the like can be employed.

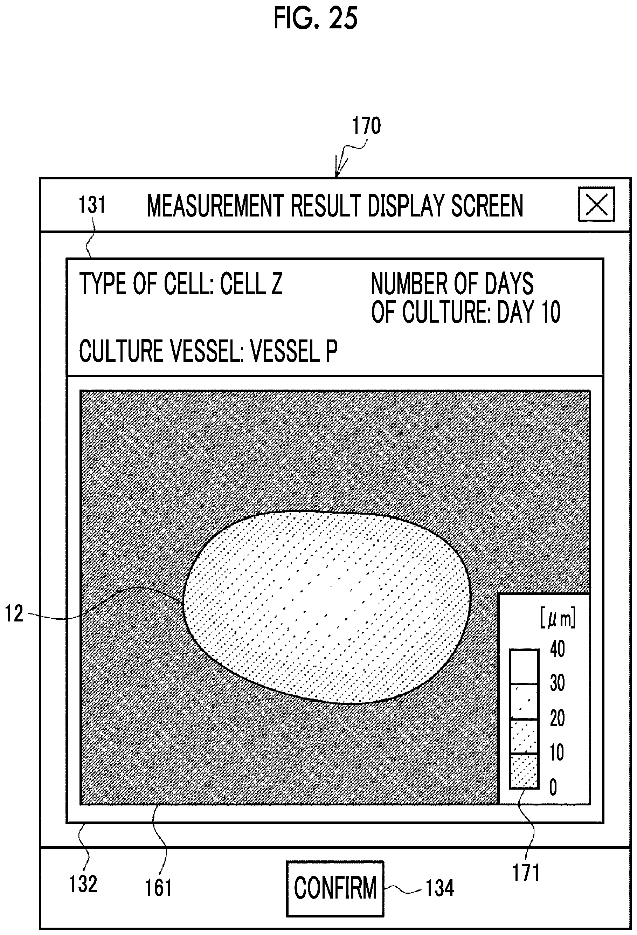

As shown in Fig. 25, in a measurement result display region 132 of a measurement result display screen 170 of the third embodiment, the reproduction image 161 is displayed instead of the three-dimensional color map 133. Then, the reproduction image 161 is colored with a color depending on the height $H(X,Y)$ of the cell 12. That is, the height calculation result 63 is displayed on the reproduction image 161 in a superimposed manner. In the measurement result display region 132, a color bar 171 indicating a correspondence relationship between the height H(X,Y) of the cell 12 and the color is displayed.

In this way, in the third embodiment, the generation unit 160 generates the reproduction image 161 from the interference fringe image 34, and the display controller 73 displays the height calculation result 63 on the reproduction image 161 in a superimposed manner. For this reason, the user can confirm the height calculation result 63 in conjunction with the reproduction image 161, and the analysis of the cell 12 is advanced.

The culture surface height-related information 81 may not necessarily be acquired. In a case where the culture surface height-related information 81 is not acquired, the height H(X,Y) + h including the culture surface height 83 is calculated as the height of the cell 12.

The shape of the object to be observed is not limited to the height H(X,Y) along the Z direction illustrated. The widths of the X direction and the Y direction may be used instead of or in addition to the height H(X,Y).

Although the four-step method has been described as an example of the phase shift method, the technique of the present disclosure is not limited thereto. A three-step method, a five-step method, a seven-step method, or the like may be used. The technique of the present disclosure is not limited to the phase shift method. A vertical scanning method that applies white light as illumination light and captures a plurality of interference fringe images 34 while moving an objective lens in the Z direction may be used. Alternatively, a method that inclines a reference plane of reference light to produce carrier fringes may be used.

The object to be observed is not limited to the cell 12 illustrated. A bacterium, a virus, or the like may be applied as the object to be observed. The object light is not limited to the object light 30 transmitted through the object to be observed, and may be object light reflected by the object to be observed. The coherent light 23 from the light source 20 may be split into light for object light and light for reference light using a beam splitter and the like. The illumination light may not be the coherent light 23, and any light may be applied as long as light produces interference fringes 33 to withstand observation.

The hardware configuration of the computer that configures the information processing apparatus 10 can be modified in various ways. For example, the information processing apparatus 10 can also be configured with a plurality of computers separated as hardware for the purpose of improvement of processing capacity and reliability. For example, the functions of the RW controller 70, the acquisition unit 71, and the display controller 73 and the function of the processing unit 72 are distributed to two computers. In this case, the information processing apparatus 10 is configured with two computers.

In this way, the hardware configuration of information processing apparatus 10 can be appropriately changed depending on required performance, such as processing capacity, safety, or reliability. Not only hardware but also an application program, such as the operation program 60, can be of course duplicated or distributed and stored in a plurality of storage devices for the purpose of ensuring safety and reliability.

In each embodiment described above, for example, as the hardware structures of processing units that execute various kinds of processing, such as the RW controller 70, the acquisition unit 71, the processing unit 72, the display controller 73, the phase difference image generation unit 100, the phase connection unit 101, the height calculation unit 102, the region extraction unit 150, and the generation unit 160, various processors described below can be used. Various processors include a programmable logic device (PLD) that is a processor capable of changing a circuit configuration after manufacture, such as a field programmable gate array (FPGA), a dedicated electric circuit that is a processor having a circuit configuration dedicatedly designed for executing specific processing, such as an application specific integrated circuit (ASIC), and the like, in addition to the CPU 52 that is a general-purpose processor configured to execute software (operation program 60) to function as various processing units, as described above.

One processing unit may be configured with one of various processors described above or may be configured with a combination of two or more processors (for example, a combination of a plurality of FPGAs and/or a combination of a CPU and an FPGA) of the same type or different types. A plurality of processing units may be configured with one processor.

As an example where a plurality of processing units are configured with one processor, first, as represented by a computer, such as a client or a server, there is a form in which one processor is configured with a combination of one or more CPUs and software, and the processor functions as a plurality of processing units. Second, as represented by system on chip (SoC) or the like, there is a form in which a processor that implements all functions of a system including a plurality of processing units into one integrated circuit (IC) chip is used. In this way, various processing units may be configured using one or more processors among various processors described above as a hardware structure.

In addition, as the hardware structure of various processors, more specifically, an electric circuit (circuitry), in which circuit elements, such as semiconductor elements, are combined, can be used.

In the technique of the present disclosure, various embodiments and various modification examples described above can also be appropriately combined. The technique of the present disclosure is not limited to the above-described embodiments, and various configurations can be of course employed without departing from the spirit and scope of the technique of the present disclosure. In addition to the program, the technique of the present disclosure extends to a storage medium that stores the program in a non-transitory manner.

The content of the above description and the content of the drawings are detailed description of portions according to the technique of the present disclosure, and are merely examples of the technique of the present disclosure. For example, the above description relating to configuration, function, operation, and advantageous effects is description relating to configuration, function, operation, and advantageous effects of the portions according to the technique of the present disclosure. Thus, it is needless to say that unnecessary portions may be deleted, new elements may be added, or replacement may be made to the content of the above description and the content of the drawings without departing from the gist of the technique of the present disclosure. Furthermore, to avoid confusion and to facilitate understanding of the portions according to the technique of the present disclosure, description relating to common technical knowledge and the like that does not require particular description to enable implementation of the technique of the present disclosure is omitted from the content of the above description and the content of the drawings.

In the specification, "A and/or B" is synonymous with "at least one of A or B". That is, "A and/or B" may refer to A alone, B alone, or a combination of A and B. Furthermore, in the specification, a similar concept to "A and/or B" applies to a case in which three or more matters are expressed by linking the matters with "and/or".

All cited documents, patent applications, and technical standards described in the specification are incorporated by reference in the specification to the same extent as in a case where each individual cited document, patent application, or technical standard is specifically and individually indicated to be incorporated by reference.

## Claims

1. An information processing apparatus that executes processing of obtaining, from an interference fringe image that is a two-dimensional distribution of intensity of interference fringes of object light as illumination light diffracted by an object to be observed and reference light without passing through the object to be observed, a phase difference image that is a two-dimensional distribution of a phase difference between the object light and the reference light, and obtaining a shape of the object to be observed based on the phase difference image, the information processing apparatus comprising:

   at least one processor configured to
   acquire object-related information regarding the object to be observed,
   read out, from a storage unit in which the object-related information and a shape profile indicating the shape of the object to be observed are stored in association with each other, the shape profile corresponding to the acquired object-related information, and
   perform phase connection with respect to the phase difference image with reference to the read-out shape profile.

2. The information processing apparatus according to claim 1,

   wherein the at least one processor is configured to
   extract a presence region of the object to be observed from the interference fringe image, and
   selectively perform the phase connection with respect to the presence region.

3. The information processing apparatus according to claim 1 or 2,
   wherein the at least one processor is configured to perform control of displaying a calculation result of the shape of the object to be observed.

4. The information processing apparatus according to claim 3,

   wherein the at least one processor is configured to
   generate a reproduction image representing any tomographic plane of the object to be observed from the interference fringe image, and
   display the calculation result of the shape of the object to be observed on the reproduction image in a superimposed manner.

5. The information processing apparatus according to any one of claims 1 to 4,
   wherein the shape of the object to be observed is a height of the object to be observed along an irradiation direction of the illumination light.

6. The information processing apparatus according to any one of claims 1 to 5,

wherein the object to be observed is a cell during culture.

7.  The information processing apparatus according to claim 6,
    wherein the at least one processor is configured to acquire culture surface height-related information regarding a height from a bottom surface to a culture surface of a culture vessel of the cell.

8.  The information processing apparatus according to claim 6 or 7,
    wherein the object-related information is a type of the cell and a culture condition of the cell.

9.  The information processing apparatus according to claim 8,
    wherein the culture condition includes at least any one of the number of days of culture, a type of a culture medium, a temperature of a culture environment, or a carbon dioxide concentration of the culture environment.

10. A method for operating an information processing apparatus that executes processing of obtaining, from an interference fringe image that is a two-dimensional distribution of intensity of interference fringes of object light as illumination light diffracted by an object to be observed and reference light without passing through the object to be observed, a phase difference image that is a two-dimensional distribution of a phase difference between the object light and the reference light, and obtaining a shape of the object to be observed based on the phase difference image, the method comprising:

    an acquisition step of acquiring object-related information regarding the object to be observed;
    a readout step of reading out, from a storage unit in which the object-related information and a shape profile indicating the shape of the object to be observed are stored in association with each other, the shape profile corresponding to the object-related information acquired in the acquisition step; and
    a phase connection step of performing phase connection with respect to the phase difference image with reference to the shape profile read out in the readout step.

11. An operation program for an information processing apparatus that executes processing of obtaining, from an interference fringe image that is a two-dimensional distribution of intensity of interference fringes of object light as illumination light diffracted by an object to be observed and reference light without passing through the object to be observed, a phase difference image that is a two-dimensional distribution of a phase difference between the object light and the reference light, and obtaining a shape of the object to be observed based on the phase difference image, the operation program causing a computer to function as:

    an acquisition unit that acquires object-related information regarding the object to be observed;
    a readout unit that reads out, from a storage unit in which the object-related information and a shape profile indicating the shape of the object to be observed are stored in association with each other, the shape profile corresponding to the object-related information acquired in the acquisition unit; and
    a phase connection unit that performs phase connection with respect to the phase difference image with reference to the shape profile read out in the readout unit.

## FIG. 1

## FIG. 2

# FIG. 3

$$I(X, Y) = A + B\cos\phi(X, Y)$$

## FIG. 4A

30

31

32

35

## FIG. 4B

30

31

32

37

## FIG. 5A

SHIFT AMOUNT 0

~34A

FIRST INTERFERENCE FRINGE IMAGE

$I\_1(X, Y) = A + B\cos\phi(X, Y)$

## FIG. 5B

SHIFT AMOUNT $\pi/2$

~34B

SECOND INTERFERENCE FRINGE IMAGE

$I\_2(X, Y) = A + B\cos\{\phi(X, Y) + (\pi/2)\}$
$= A - B\sin\phi(X, Y)$

## FIG. 5C

SHIFT AMOUNT $\pi$

~34C

THIRD INTERFERENCE FRINGE IMAGE

$I\_3(X, Y) = A + B\cos\{\phi(X, Y) + \pi\}$
$= A - B\cos\phi(X, Y)$

## FIG. 5D

SHIFT AMOUNT $3\pi/2$

~34D

FOURTH INTERFERENCE FRINGE IMAGE

$I\_4(X, Y) = A + B\cos\{\phi(X, Y) + (3\pi/2)\}$
$= A + B\sin\phi(X, Y)$

# FIG. 6

$$\frac{I\_4(X, Y) - I\_2(X, Y)}{I\_1(X, Y) - I\_3(X, Y)} = \frac{A + B\sin\phi(X, Y) - A + B\sin\phi(X, Y)}{A + B\cos\phi(X, Y) - A + B\cos\phi(X, Y)}$$

$$= \tan\phi(X, Y)$$

$$\phi(X, Y) = \arctan\frac{I\_4(X, Y) - I\_2(X, Y)}{I\_1(X, Y) - I\_3(X, Y)}$$

40

# FIG. 7

$$H(X, Y) = \frac{\lambda \, \phi(X, Y)}{4\pi} - h$$

# FIG. 8

## FIG. 9

# FIG. 10

90

| INFORMATION INPUT SCREEN | ☒ |
|---|---|

PLEASE INPUT INFORMATION REGARDING CELL TO BE MEASURED.

TYPE OF CELL     | CELL A | ∨ | ～91

NUMBER OF DAYS OF CULTURE | DAY 1 | ∨ | ～92

TYPE OF CULTURE VESSEL | VESSEL A | ∨ | ～93

OK ～94

**OBJECT-RELATED INFORMATION**

80～

TYPE OF CELL: CELL A
NUMBER OF DAYS OF CULTURE: DAY 1

**CULTURE SURFACE HEIGHT-RELATED INFORMATION**

81～

TYPE OF CULTURE VESSEL: VESSEL A

# FIG. 11

SHAPE PROFILE TABLE

| TYPE OF CELL | NUMBER OF DAYS OF CULTURE | SHAPE PROFILE |
|---|---|---|
| CELL A | DAY 1 | |
| | DAY 2 | |
| | DAY 3 | |
| CELL B | DAY 1 | |
| | DAY 2 | |
| ⋮ | | |

# FIG. 12

**CULTURE SURFACE HEIGHT TABLE** — 62

| TYPE OF CULTURE VESSEL | CULTURE SURFACE HEIGHT |
|---|---|
| VESSEL A | 3 mm |
| VESSEL B | 5 mm |
| VESSEL C | 2.5 mm |
| ⋮ | |

— 83

# FIG. 13

**PROCESSING UNIT** — 72

PHASE DIFFERENCE IMAGE GENERATION UNIT — 100

↓

PHASE CONNECTION UNIT — 101

↓

HEIGHT CALCULATION UNIT — 102

# FIG. 14

| FIRST INTERFERENCE FRINGE IMAGE | SECOND INTERFERENCE FRINGE IMAGE | THIRD INTERFERENCE FRINGE IMAGE | FOURTH INTERFERENCE FRINGE IMAGE |
|---|---|---|---|
| 34A | 34B | 34C | 34D |

PHASE DIFFERENCE IMAGE GENERATION UNIT ~100

X

40 — PHASE DIFFERENCE IMAGE

Y

Ys

$\phi(X, Ys)$

X

82

SHAPE PROFILE → PHASE CONNECTION UNIT ~101

X

40P — PHASE DIFFERENCE IMAGE

Y

Ys

$\phi(X, Ys)$

X

TO HEIGHT CALCULATION UNIT

# FIG. 15

$$\phi E = \phi S + \Sigma \Delta \phi$$

$$-\pi < \phi j - \phi i \leq \pi \longrightarrow \Delta \phi = \phi j - \phi i$$

$$\phi j - \phi i \leq -\pi \longrightarrow \Delta \phi = \phi j - \phi i + 2\pi$$

$$\phi j - \phi i > \pi \longrightarrow \Delta \phi = \phi j - \phi i - 2\pi$$

# FIG. 16

# FIG. 17

40P — PHASE DIFFERENCE IMAGE

FROM PHASE
CONNECTION UNIT

83

CULTURE
SURFACE HEIGHT

HEIGHT
CALCULATION
UNIT — 102

63 — HEIGHT
CALCULATION RESULT

H (X, Y)

TO RW CONTROLLER

# FIG. 18

130

131 | MEASUREMENT RESULT DISPLAY SCREEN | ☒

TYPE OF CELL: CELL Z

NUMBER OF DAYS
OF CULTURE: DAY 10

CULTURE VESSEL: VESSEL P

H [$\mu$m]
50
40
30
20
10
0
0

12

133

300

X [$\mu$m]

Y [$\mu$m]

13B

300    0

132

CONFIRM — 134

# FIG. 19

START

ST100 —
ACQUIRE OBJECT-RELATED INFORMATION
AND CULTURE SURFACE HEIGHT-RELATED INFORMATION

ST110 —
READ OUT SHAPE PROFILE AND CULTURE SURFACE HEIGHT

ST120 —
STORE INTERFERENCE FRINGE IMAGE

ST130 —
READ OUT INTERFERENCE FRINGE IMAGE

ST140 —
GENERATE PHASE DIFFERENCE IMAGE

ST150 —
PERFORM PHASE CONNECTION WITH REFERENCE TO SHAPE PROFILE

ST160 —
CALCULATE HEIGHT OF CELL

ST170 —
DISPLAY MEASUREMENT RESULT DISPLAY SCREEN

END

# FIG. 20A

⟨CASE WHERE SHAPE PROFILE IS NOT REFERRED TO⟩

| PROCESSING 1 | PROCESSING 2 | PROCESSING 3 | · · · | PROCESSING M-1 | PROCESSING M |

EXECUTE PROCESSING COMPREHENSIVELY

# FIG. 20B

⟨CASE WHERE SHAPE PROFILE IS REFERRED TO⟩

| PROCESSING 1 | PROCESSING 2 | PROCESSING 3 |

SELECTIVELY EXECUTE PROCESSING
DEPENDING ON SHAPE PROFILE

# FIG. 20C

⟨CASE WHERE SHAPE PROFILE IS REFERRED TO⟩

RANK 1     RANK 2     RANK 3

| PROCESSING 1 | PROCESSING 2 | PROCESSING 3 | · · · |

EXECUTE PROCESSING BASED ON PRIORITY DETERMINED
DEPENDING ON SHAPE PROFILE

# FIG. 21

140 —

| OBJECT-RELATED INFORMATION |
|---|

TYPE OF CELL: CELL S    NUMBER OF DAYS OF CULTURE: DAY 5

TYPE OF CULTURE: CULTURE D

TEMPERATURE: 36 °C    CARBON DIOXIDE CONCENTRATION: 5%

# FIG. 22

REGION EXTRACTION UNIT ~150

151

CP

CP

34

33

151

CP

151

SELECTIVELY PERFORM PHASE CONNECTION WITH RESPECT TO PRESENCE REGION

PHASE CONNECTION UNIT ~101

# FIG. 23

```
┌─────────────────────┐
│   INTERFERENCE      │ ～34
│   FRINGE IMAGE      │
└─────────────────────┘
          │
          ▼
┌─────────────────────┐
│   GENERATION        │ ～160
│   UNIT              │
└─────────────────────┘
          │
          ▼
┌─────────────────────┐
│   REPRODUCTION      │ ～161
│   IMAGE             │
└─────────────────────┘
          │
          ▼
    TO DISPLAY
    CONTROLLER
```

# FIG. 24

EP 4 092 376 A1

# FIG. 25

170

## MEASUREMENT RESULT DISPLAY SCREEN    ☒

131

TYPE OF CELL: CELL Z          NUMBER OF DAYS
                              OF CULTURE: DAY 10

CULTURE VESSEL: VESSEL P

12

[μm]
40
30
20
10
0
171

132    161                   CONFIRM ～134

## INTERNATIONAL SEARCH REPORT

| | International application No. |
|---|---|
| | PCT/JP2020/038539 |

**A. CLASSIFICATION OF SUBJECT MATTER**
G01B 9/02(2006.01)i; G01B 11/24(2006.01)i; C12M 1/34(2006.01)i
FI: G01B11/24 D; C12M1/34 A; G01B9/02

According to International Patent Classification (IPC) or to both national classification and IPC

**B. FIELDS SEARCHED**

Minimum documentation searched (classification system followed by classification symbols)
G01B9/00-9/10; G01B11/00-11/30

| Documentation searched other than minimum documentation to the extent that such documents are included in the fields searched | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2006-284186 A (LASERTEC CORPORATION) 19 October 2006 (2006-10-19) paragraphs [0081]-[0083] | 1-11 |
| A | JP 2007-54727 A (MAZDA MOTOR CORPORATION) 08 March 2007 (2007-03-08) paragraphs [0031]-[0033] | 1-11 |
| A | JP 2018-4279 A (KEYENCE CORP.) 11 January 2018 (2018-01-11) paragraphs [0207]-[0208] | 1-11 |

☐ Further documents are listed in the continuation of Box C.    ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 13 November 2020 (13.11.2020) | 08 December 2020 (08.12.2020) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

| Patent Documents referred in the Report | Publication Date | Patent Family | Publication Date |
|---|---|---|---|
| JP 2006-284186 A | 19 Oct. 2006 | (Family: none) | |
| JP 2007-54727 A | 08 Mar. 2007 | (Family: none) | |
| JP 2018-4279 A | 11 Jan. 2018 | US 2017/0370708 A1 paragraphs [0273]-[0274] | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006284186 A